# EUROPEAN PATENT APPLICATION

(11) **EP 2 979 606 A1**
(43) Date of publication of application: **03.02.2016**
(21) Application number: 13880187.3
(22) Date of filing: 25.09.2013
(51) Int. Cl.: A61B 1/00, A61B 1/04, G02B 23/24, G06T 1/00

(54) **IMAGE PROCESSING DEVICE, ENDOSCOPIC DEVICE, PROGRAM AND IMAGE PROCESSING METHOD**

(30) Priority: 27.03.2013 JP 2013067423
(71) Applicant: Olympus Corporation, Shibuya-ku Tokyo 151-0072 (JP)
(72) Inventor: ROKUTANDA, Etsuko, Tokyo 151-0072 (JP)
(74) Representative: Gunzelmann, Rainer
(86) International application number: PCT/JP2013/075869
(87) International publication number: WO 2014/155782

(57) **Abstract**

An image processing device includes an image acquisition section (305) that acquires a captured image that includes an image of the object, a distance information acquisition section (340) that acquires distance information based on the distance from an imaging section (200) to the object when the imaging section (200) captured the captured image, an in-focus determination section (370) that determines whether or not the object is in focus within a pixel or an area within the captured image based on the distance information, a classification section (310) that performs a classification process that classifies the structure of the object, and controls the target of the classification process corresponding to the results of the determination as to whether or not the object is in focus within the pixel or the area, and an enhancement processing section (330) that performs an enhancement process on the captured image based on the results of the classification process.

## Description

### TECHNICAL FIELD

The present invention relates to an image processing device, an endoscope apparatus, a program, an image processing method, and the like.

### BACKGROUND ART

An improvement in the detection accuracy of a lesion inside a body cavity has been desired in the field of endoscopic diagnosis. An endoscope that includes a zoom optical system that improves the detection accuracy by magnifying the difference in tissue between a lesion area and a normal area at a magnification almost equal to that of a microscope (hereinafter referred to as "zoom endoscope") has been known.

A zoom endoscope may achieve a magnification of several ten to several hundred times. The microstructure of a mucous membrane surface layer can be observed by utilizing such a zoom endoscope in combination with a method that enhances the contrast by spraying a dye. It is known that a lesion area and a normal area differ in pattern, and such a difference in pattern has been used as a lesion diagnostic criterion.

Attempts have been made to display the structure of the surface area of a mucous membrane in a state in which the contrast of the structure is improved by image processing without spraying a dye. For example, Patent Document 1 discloses a method that compares the luminance level of an attention pixel (pixel in question) in a locally extracted area with the luminance level of its peripheral pixels, and colors the attention area (area in question) when the attention area is darker than its peripheral area. The method disclosed in Patent Document 1 is based on the assumption that a distant object is captured as dark since the intensity of reflected light from the surface of tissue decreases.

An image that prevents a situation in which a lesion is missed, and improves the accuracy of qualitative diagnosis may be provided by selectively enhancing a lesion (selectively displaying a lesion in an enhanced state). For example, Patent Document 2 discloses a method that classifies an image obtained by capturing tissue through a grid division process and a feature quantity extraction process, and performs a different display process corresponding to each classification.

### RELATED-ART DOCUMENT

### PATENT DOCUMENT

Patent Document 1: JP-A-2003-088498

Patent Document 2: JP-A-2011-215680

### SUMMARY OF THE INVENTION

### TECHNICAL PROBLEM

When performing zoom observation using an endoscope apparatus, the depth of field significantly decreases due to an increase in magnification as compared with normal observation. Specifically, an image captured during zoom observation occasion may include an area in which the object is in focus, and an area in which the object is out of focus. In such a case, it is difficult to accurately enhance a legion in an area in which the object is out of focus since the amount of information obtained from the image is insufficient.

For example, a ductal structure (referred to as "pit pattern") observed on the surface of tissue may be enhanced by performing a matching process on a known pit pattern shape and the captured image to classify the pit pattern within the image, and displaying the classification results. However, it may be impossible to detect the pit pattern by the matching process in an area of the image in which the object is out of focus (i.e., incorrect classification results may be obtained), and the reliability of display may deteriorate.

Several aspects of the invention may provide an image processing device, an endoscope apparatus, a program, an image processing method, and the like that can improve the reliability of enhancement display.

### SOLUTION TO PROBLEM

According to one aspect of the invention, there is provided an image processing device comprising:
an image acquisition section that acquires a captured image that includes an image of an object;
a distance information acquisition section that acquires distance information based on a distance from an imaging section to the object when the imaging section captured the captured image;
an in-focus determination section that determines whether or not the object is in focus within a pixel or an area within the captured image based on the distance information;
a classification section that performs a classification process that classifies a structure of the object, and controls a target of the classification process corresponding to results of the determination as to whether or not the object is in focus within the pixel or the area; and
an enhancement processing section that performs an enhancement process on the captured image based on results of the classification process.

According to one aspect of the invention, the classification process that classifies the structure of the object is performed, and the target of the classification process is controlled corresponding to the results of the determination as to whether or not the object is in focus within each pixel or each area within the captured image. The enhancement process is performed based on the results of the classification process. This makes it possible to improve the reliability of enhancement display.

According to another aspect of the invention, there is provided an endoscope apparatus comprising the above image processing device.

According to another aspect of the invention, there is provided a program that causes a computer to perform steps of:
acquiring a captured image that includes an image of an object;
acquiring distance information based on a distance from an imaging section to the object when the imaging section captured the captured image;
determining whether or not the object is in focus within a pixel or an area within the captured image based on the distance information;
performing a classification process that classifies a structure of the object, and controlling a target of the classification process corresponding to results of the determination as to whether or not the object is in focus within the pixel or the area; and
performing an enhancement process on the captured image based on results of the classification process.

According to another aspect of the invention, there is provided an image processing method comprising:
acquiring a captured image that includes an image of an object;
acquiring distance information based on a distance from an imaging section to the object when the imaging section captured the captured image;
determining whether or not the object is in focus within a pixel or an area within the captured image based on the distance information;
performing a classification process that classifies a structure of the object, and controlling a target of the classification process corresponding to results of the determination as to whether or not the object is in focus within the pixel or the area; and
performing an enhancement process on the captured image based on results of the classification process.

### BRIEF DESCRIPTION OF DRAWINGS

FIG. 1A illustrates the relationship between an imaging section and the object when observing an abnormal part, and FIG. 1B illustrates an example of the acquired image.
FIG. 2 illustrates a configuration example of an image processing device.
FIG. 3 illustrates a configuration example of an endoscope apparatus (first embodiment).
FIG. 4 illustrates a configuration example of an external I/F section (first embodiment).
FIG. 5 is a view illustrating a change in the depth of field of an imaging system when a zoom lever is operated.
FIG. 6 illustrates a detailed configuration example of an image processing section.
FIG. 7 illustrates a detailed configuration example of an in-focus determination section (first embodiment).
FIG. 8 is a view illustrating a classification process.
FIG. 9 illustrates a configuration example of an endoscope apparatus (second embodiment).
FIG. 10 illustrates a configuration example of an external I/F section (second embodiment).
FIG. 11 illustrates a detailed configuration example of a focus control section.
FIG. 12 illustrates a detailed configuration example of an in-focus determination section (second embodiment).
FIG. 13 is a view illustrating a classification process (second embodiment).
FIG. 14 illustrates a detailed configuration example of a classification section.
FIGS. 15A and 15B are views illustrating a process performed by a surface shape calculation section.
FIG. 16A illustrates an example of a basic pit, and FIG. 16B illustrates an example of a corrected pit.
FIG. 17 illustrates a detailed configuration example of a surface shape calculation section.
FIG. 18 illustrates a detailed configuration example of a classification processing section when implementing a first classification method.
FIGS. 19A to 19F are views illustrating a specific example of a classification process.
FIG. 20 illustrates a detailed configuration example of a classification processing section when implementing a second classification method.
FIG. 21 illustrates an example of a classification type when a plurality of classification types are used.
FIGS. 22A to 22F illustrate an example of a pit pattern.

### DESCRIPTION OF EMBODIMENTS

Exemplary embodiments of the invention are described below. Note that the following exemplary embodiments do not in any way limit the scope of the invention laid out in the claims. Note also that all of the elements described in connection with the following exemplary embodiments should not necessarily be taken as essential elements of the invention.

### 1. Outline

An outline of several embodiments of the invention is described below taking an example in which an endoscope apparatus performs a pit pattern classification process.

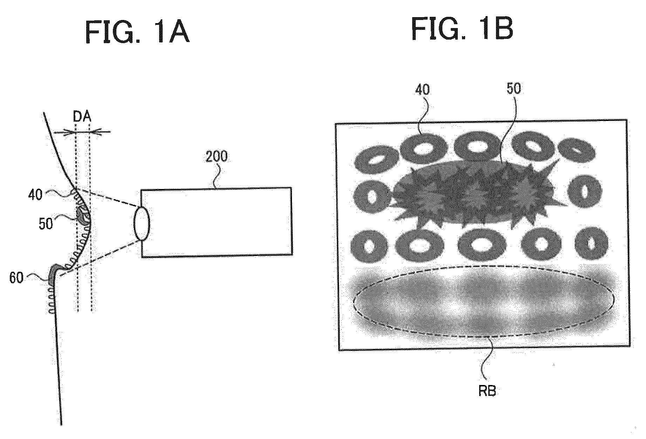

FIG. 1A illustrates the relationship between an imaging section 200 and the object when observing an abnormal part (e.g., early lesion). FIG. 1B illustrates an example of an image acquired when observing the abnormal part. A normal duct 40 represents a normal pit pattern, an abnormal duct 50 represents an abnormal pit pattern having an irregular shape, and a duct disappearance area 60 represents an abnormal area in which the pit pattern has disappeared due to a lesion.

When the operator (user) has found an abnormal part (abnormal duct 50 and duct disappearance area 60) (see FIG. 1A), the operator brings the imaging section 200 closer to the abnormal part so that the imaging section 200 directly faces the abnormal part as much as possible. As illustrated in FIG. 1B, a normal part (normal duct 40) has a pit pattern in which regular structures are uniformly arranged.

According to several embodiments of the invention, such a normal part is detected by image processing by registering or learning a normal pit pattern structure as known characteristic information (prior information), and performing a matching process or the like. An area in which the normal pit pattern has not been detected is classified as an abnormal part in which the pit pattern has an irregular shape, or has disappeared, for example. It is possible to prevent a situation in which an abnormal part is missed, and improve the accuracy of qualitative diagnosis by thus classifying the pit pattern as a normal part or an abnormal part, and enhancing the classification results.

When performing the classification process using the matching process or the like, however, erroneous classification may occur in an area of the image in which the amount of information is small. Specifically, the depth of field DA is very shallow (e.g., several mm) when performing zoom observation in a state in which the imaging section 200 is brought close to the object (see FIG. 1A). Therefore, an out-of-focus area RB easily occurs within the image (see FIG. 1B). Since the accuracy of the matching process decreases in the area RB, an area that should be classified as a normal part may be classified (displayed) as an abnormal part.

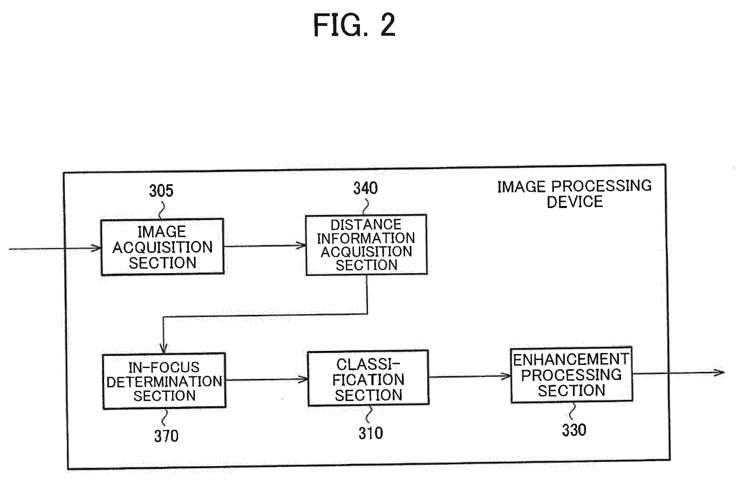

An image processing device according to several embodiments of the invention includes an image acquisition section 305 that acquires a captured image that includes an image of the object, a distance information acquisition section 340 that acquires distance information based on the distance from the imaging section 200 to the object when the imaging section 200 captured the captured image, an in-focus determination section 370 that determines whether or not the object is in focus within a pixel or an area within the captured image based on the distance information, a classification section 310 that performs a classification process that classifies the structure of the object, and controls the target of the classification process corresponding to the results of the determination as to whether or not the object is in focus within the pixel or the area, and an enhancement processing section 330 that performs an enhancement process on the captured image based on the results of the classification process (see FIG. 2).

According to this configuration, the area RB which lies outside the depth of field and for which the reliability of the classification results decreases can be detected by locally determining whether the object is in focus or out of focus. It is possible to perform the enhancement (display) process based on highly reliable classification results by performing the classification process based on the detection results.

For example, the classification section 310 controls the target of the classification process by excluding the pixel or the area for which it has been determined that the object is out of focus from the target of the matching process, and classifying the pixel or the area (for which it has been determined that the object is out of focus) as "unknown" (that represents that it is unknown whether the pit pattern should be classified as a normal part or an abnormal part). Alternatively, the classification section 310 performs the matching process regardless of the results as to whether or not the object is in focus, and classifies the pixel or the area for which it has been determined that the object is out of focus as "unknown". It is possible to prevent erroneous display due to a decrease in the accuracy of the matching process by thus performing the classification process based on the results of the in-focus determination process.

The term "distance information" used herein refers to information that links each position of the captured image to the distance to the object at each position of the captured image. For example, the distance information is a distance map in which the distance to the object in the optical axis direction of the imaging section 200 is linked to each pixel. Note that the distance information is not limited to the distance map, but may be various types of information that are acquired based on the distance from the imaging section 200 to the object (described later).

The classification process is not limited to the pit pattern classification process. The term "classification process" used herein refers to an arbitrary process that classifies the structure of the object corresponding to the type, the state, or the like of the structure. The term "structure" used herein in connection with the object refers to a structure that can assist the user in observation and diagnosis when the classification results are presented to the user. For example, when the endoscope apparatus is a medical endoscope apparatus, the structure may be a pit pattern, a polyp that projects from a mucous membrane, the folds of the digestive tract, a blood vessel, or a lesion (e.g., cancer). The classification process classifies the structure of the object corresponding to the type, the state (e.g., normal/abnormal), or the degree of abnormality of the structure.

Note that the classification process may be implemented in various ways. For example, the classification process may calculate the shape of the surface of the object from the distance information, perform a matching process on a reference pit pattern (that has been deformed corresponding to the shape of the surface of the object) and the image, and classify the pit pattern within the image based on the matching results (described later). Alternatively, the classification process may perform a matching process on the reference pit pattern and the image using a phase-only correction (POC) process or the like without deforming the reference pit pattern using the distance information, and classify the pit pattern based on the matching results.

The object may be classified by extracting a specific structure (e.g., polyp or groove). For example, a stereo matching process is performed on a stereo image to acquire a distance map, and a low-pass filtering process, a morphological process, or the like is performed on the distance map to acquire global shape information about the object. The global shape information is subtracted from the distance map to acquire information about a local concave-convex structure. The known characteristic information (e.g., the size and the shape of a specific polyp, or the depth and the width of a groove specific to a lesion) about the classification target structure is compared with the information about a local concave-convex structure to extract a concave-convex structure that agrees with the known characteristic information. A specific structure (e.g., polyp or groove) can thus be classified (detected).

The term "enhancement process" used herein refers to a process that enhances or differentiates a specific target within the image. For example, the enhancement process may be a process that enhances the structure, the color, or the like of an area that has been classified as a specific type or a specific state, or may be a process that highlights such an area, or may be a process that encloses such an area with a line, or may be a process that adds a mark that represents such an area. A specific area may be caused to stand out (or be differentiated) by performing the above process on an area other than the specific area.

### 2. First embodiment

### 2.1. Endoscope apparatus

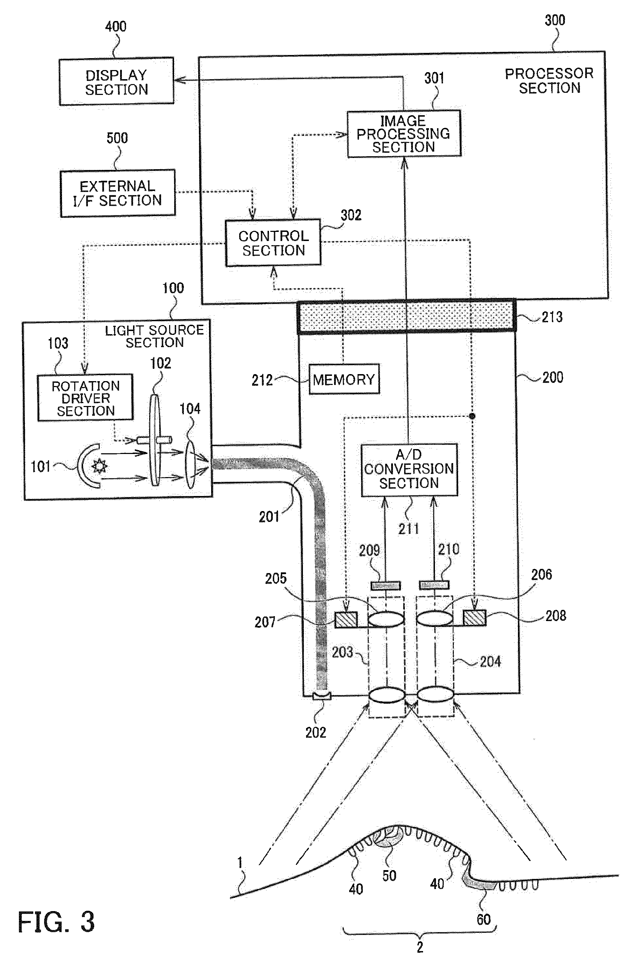

FIG. 3 illustrates a configuration example of an endoscope apparatus according to a first embodiment. The endoscope apparatus includes a light source section 100, an imaging section 200, a processor section 300 (control device), a display section 400, and an external I/F section 500.

The light source section 100 includes a white light source 101, a rotary color filter 102 that includes a plurality of color filters that differ in spectral transmittance, a rotation driver section 103 that drives the rotary color filter 102, and a condenser lens 104 that focuses light (that has passed through the rotary color filter 102 and has spectral characteristics) on the incident end face of a light guide fiber 201.

The rotary color filter 102 includes a red color filter, a green color filter, a blue color filter, and a rotary motor.

The rotation driver section 103 rotates the rotary color filter 102 at a given rotational speed in synchronization with the imaging period of an image sensor 209 and an image sensor 210 based on a control signal output from a control section 302 included in the processor section 300. For example, when the rotary color filter 102 is rotated at 20 revolutions per second, each color filter crosses the incident white light every 1/60th of a second. In this case, the image sensor 209 and the image sensor 210 capture the reflected light from the observation target to which each color light (R, G, or B) has been applied, and transfer the resulting image every 1/60th of a second. Specifically, the endoscope apparatus according to the first embodiment frame-sequentially captures an R image, a G image, and a B image every 1/60th of a second, and the substantial frame rate is 20 fps.

The imaging section 200 is formed to be elongated and flexible so that the imaging section 200 can be inserted into a body cavity (e.g., stomach or large intestine), for example. The imaging section 200 includes the light guide fiber 201 that guides the light focused by the light source section 100, an illumination lens 202 that diffuses the light guided by the light guide fiber 201, and applies the diffused light to the observation target, and an objective lens system 203 and an objective lens system 204 that focus the reflected light from the observation target. The objective lens system 203 includes a zoom lens 205 that adjusts the optical magnification, and the objective lens system 204 includes a zoom lens 206 that adjusts the optical magnification. The imaging section 200 also includes a zoom lens driver section 207 that drives the zoom lens 205, a zoom lens driver section 208 that drives the zoom lens 206, the image sensor 209 that detects the light focused by the objective lens system 203, the image sensor 210 that detects the light focused by the objective lens system 204, and an A/D conversion section 211 that converts analog signals photoelectrically converted by the image sensor 209 and the image sensor 210 into digital signals. The imaging section 200 further includes a memory 212 that stores scope ID information and specific information (including production variations) about the imaging section 200, and a connector 213 that is removably connected to the processor section 300.

The zoom lens driver section 207 and the zoom lens driver section 208 are connected to the external I/F section 500 and the control section 302, and control the zoom lens position according to information input to the external I/F section 500. The zoom lens driver section 207 and the zoom lens driver section 208 are implemented by a voice coil motor (VCM), for example. The image sensor 209 and the image sensor 210 are monochrome single-chip image sensors, for example. A CCD image sensor, a CMOS image sensor, or the like may be used as the image sensor 209 and the image sensor 210.

The objective lens system 203 and the objective lens system 204 are disposed at a given interval so that a given parallax image (hereinafter referred to as "stereo image") can be captured. The objective lens system 203 and the objective lens system 204 respectively form a left image and a right image on the image sensor 209 and the image sensor 210. The A/D conversion section 211 converts the left image output from the image sensor 209 and the right image output from the image sensor 210 into digital signals, and outputs the resulting left image and the resulting right image to an image processing section 301. The memory 212 is connected to the control section 302, and transmits the scope ID information and the specific information (including production variations) to the control section 302.

The processor section 300 includes the image processing section 301 (corresponding to an image processing device) that performs various types of image processing on the image transmitted from the A/D conversion section 211, and the control section 302 that controls each section of the endoscope apparatus.

The display section 400 displays the image transmitted from the image processing section 301. The display section 400 is a display device (e.g., CRT or liquid crystal monitor) that can display a moving image (movie (video)).

The external I/F section 500 is an interface that allows the user to input information and the like to the endoscope apparatus. For example, the external I/F section 500 includes a power switch (power ON/OFF switch), a shutter button (capture start button), a mode (e.g., imaging mode) switch (e.g., a switch for selectively enhancing the structure of the surface of tissue), and the like. The external I/F section 500 outputs the input information to the control section 302.

### 2.2. Observation mode and depth of field

The relationship between the zoom lens 205 and the zoom lens 206 included in the imaging section 200 and the external I/F section 500 is described in detail below. The endoscope apparatus according to the first embodiment can implement two observation modes that differ in observation magnification.

Specifically, the endoscope apparatus according to the first embodiment can implement a normal observation mode and a zoom observation mode. In the normal observation mode, screening observation is mainly performed using a deep-focus wide-field image. In the zoom observation mode, the mucosal membrane structure, the blood vessel distribution, and the like included in a lesion found by screening observation are closely observed to determine whether or not the lesion is malignant.

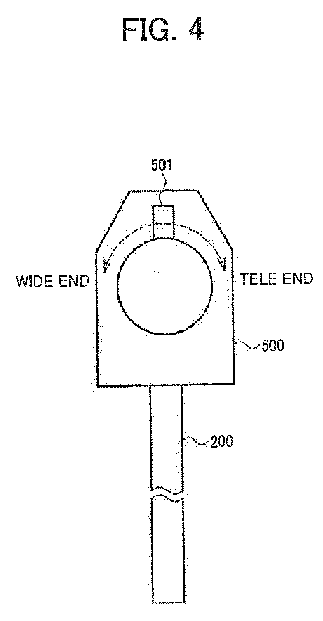

FIG. 4 illustrates a configuration example of the external I/F section 500 according to the first embodiment. The observation mode is automatically switched between the normal observation mode and the zoom observation mode when the user has operated a zoom lever 501 illustrated in FIG. 4. Specifically, the user sets (turns) the zoom lever 501 to the WIDE end when the user desires to perform screening observation, and turns the zoom lever 501 toward the TELE end to change the zoom magnification stepwise when the user desires to perform zoom observation.

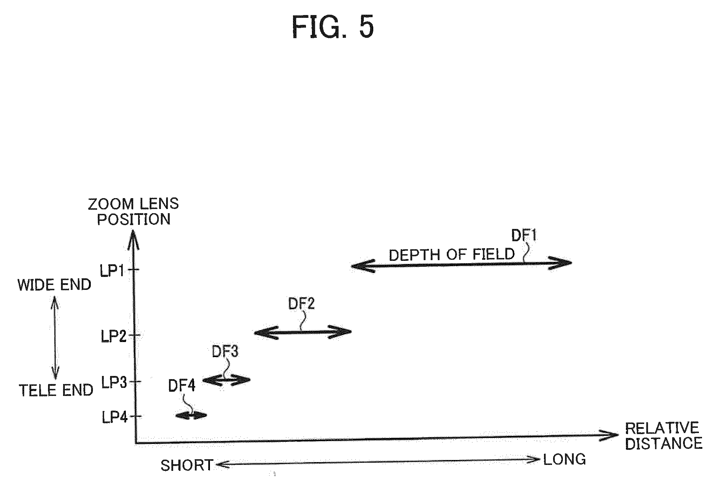

FIG. 5 is a view illustrating a change in the depth of field of an imaging system that occurs when the zoom lever 501 is operated. The imaging system includes the objective lens system 203 (that includes the zoom lens 205) and the image sensor 209. The following description similarly applies to the imaging system that includes the objective lens system 204 (that includes the zoom lens 206) and the image sensor 210.

As illustrated in FIG. 5, when the zoom lever 501 has been set to the WIDE end, the zoom lens 205 is set to a position LP 1 that corresponds to a wide viewing angle. When the zoom lever 501 has been set to the WIDE end, the longest in-focus distance and the deepest depth of field DF1 are achieved so that the relative distance with respect to the object that is considered to be used during screening observation falls within the depth of field DF1. The zoom lens 205 is set to positions LP2 to LP4 by moving the zoom lever 501 toward the TELE end stepwise (e.g., in five steps). In this case, the viewing angle and the in-focus distance decrease, and the depth of field (DF2 to DF4) becomes shallow as the zoom lever 501 is moved closer to the TELE end. The depth of field is shallow when the zoom lever 501 has been set to the TELE end, but the object can be observed more closely (i.e., high-magnification zoom observation can be performed).

### 2.3. Image processing device

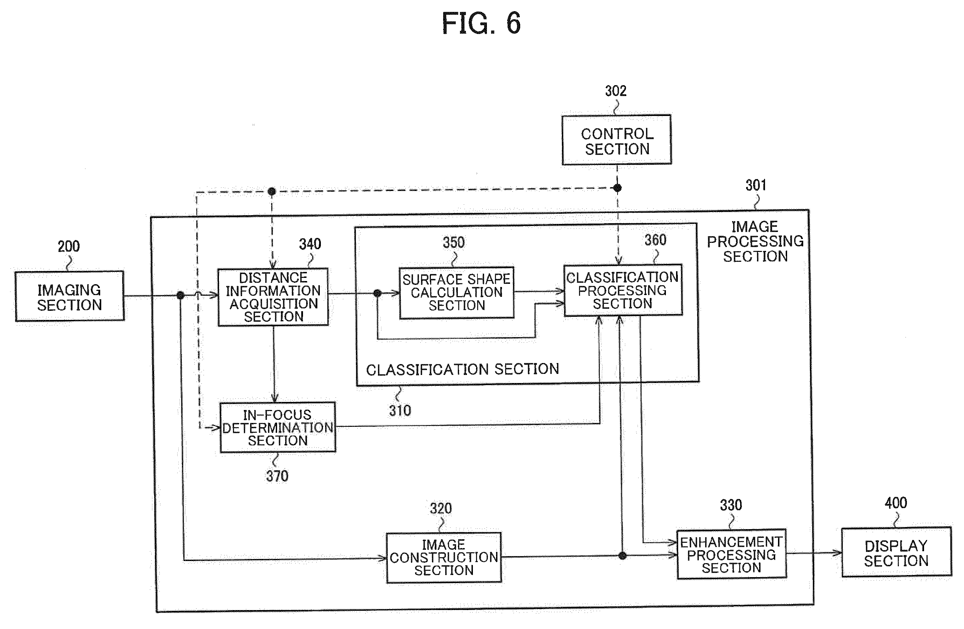

FIG. 6 illustrates a detailed configuration example of the image processing section 301 according to the first embodiment. The image processing section 301 includes a classification section 310, an image construction section 320, an enhancement processing section 330, a distance information acquisition section 340 (distance map calculation section), and an in-focus determination section 370. Although an example in which the pit pattern classification process is performed by utilizing the matching process is described below, various other classification processes may also be used (see above).

The distance information acquisition section 340 acquires the stereo image output from the A/D conversion section 211, and acquires the distance information based on the stereo image. Specifically, the distance information acquisition section 340 performs a matching calculation process on the left image (reference image) and a local area of the right image along an epipolar line that passes through the attention pixel situated at the center of a local area of the left image to calculate a position at which the maximum correlation is obtained as a parallax. The distance information acquisition section 340 converts the calculated parallax into the distance in the Z-axis direction to acquire the distance information (e.g., distance map), and outputs the distance information to the in-focus determination section 370 and the classification section 310.

The term "distance information" used herein refers to various types of information that are acquired based on the distance from the imaging section 200 to the object. For example, when implementing triangulation using a stereo optical system, the distance with respect to an arbitrary point of a plane that connects two lenses that produce a parallax may be used as the distance information. Alternatively, the distance information may be acquired using a Time-of-Flight method. When using a Time-of-Flight method, a laser beam or the like is applied to the object, and the distance is measured based on the time of arrival of the reflected light. In this case, the distance with respect to the position of each pixel of the plane of the image sensor that captures the reflected light may be acquired as the distance information, for example. Although an example in which the distance measurement reference point is set to the imaging section 200 has been described above, the reference point may be set at an arbitrary position other than the imaging section 200. For example, the reference point may be set at an arbitrary position within a three-dimensional space that includes the imaging section 200 and the object. The distance information acquired using such a reference point is also included within the scope of the term "distance information".

The distance from the imaging section 200 to the object may be the distance from the imaging section 200 to the object in the depth direction, for example. For example, the distance from the imaging section 200 to the object in the direction of the optical axis of the imaging section 200 may be used. Specifically, the distance to a given point of the object is the distance from the imaging section 200 to the object along a line that passes through the given point and is parallel to the optical axis. Examples of the distance information include a distance map. The term "distance map" used herein refers to a map in which the distance (depth) to the object in the Z-axis direction (i.e., the direction of the optical axis of the imaging section 200) is specified for each point in the XY plane (e.g., each pixel of the captured image), for example.

The distance information acquisition section 340 may set a virtual reference point at a position that can maintain a relationship similar to the relationship between the distance values of the pixels on the distance map acquired when the reference point is set to the imaging section 200, to acquire the distance information based on the distance from the imaging section 200 to each corresponding point. For example, when the actual distances from the imaging section 200 to three corresponding points are respectively "3", "4", and "5", the distance information acquisition section 340 may acquire distance information "1.5", "2", and "2.5" respectively obtained by halving the actual distances "3", "4", and "5" while maintaining the relationship between the distance values of the pixels.

The image construction section 320 acquires the stereo image (left image and right image) output from the A/D conversion section 211, and performs image processing (e.g., OB process, gain process, and gamma process) on the stereo image to generate an image that can be output from (displayed on) the display section 400. The image construction section 320 outputs the generated image to the classification section 310 and the enhancement processing section 330.

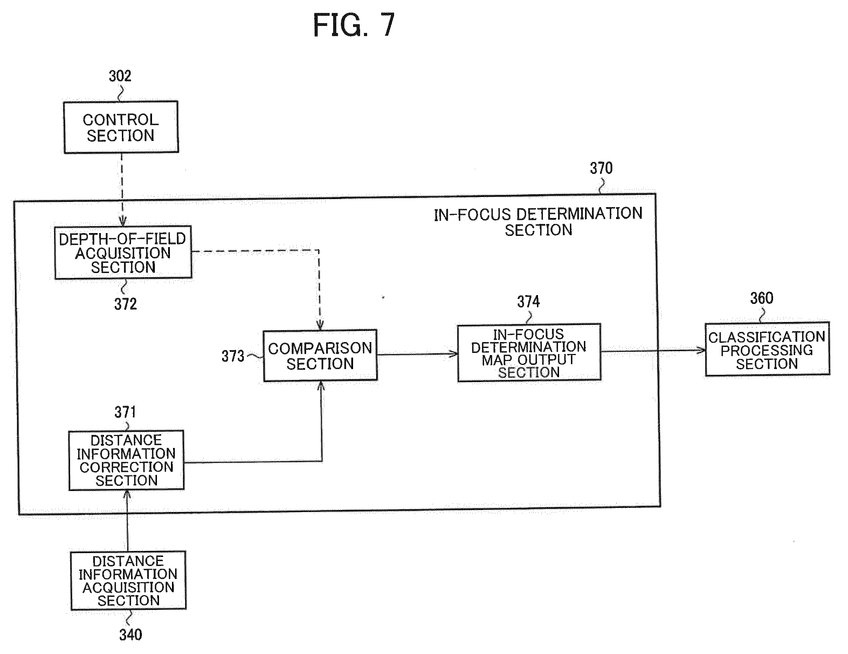

The in-focus determination section 370 performs the in-focus determination process corresponding to each pixel or each area (e.g., each area when the captured image is divided into a plurality of areas having a given size) within the captured image by comparing the distance from the imaging section 200 to the object with the depth of field of the imaging section 200. FIG. 7 illustrates a detailed configuration example of the in-focus determination section 370. The in-focus determination section 370 includes a distance information correction section 371 (distance map correction section), a depth-of-field acquisition section 372, a comparison section 373, and an in-focus determination map output section 374. Note that an example when the distance information is the distance map is described below.

The distance information correction section 371 performs a low-pass filtering process using a given size (N×N pixels) on the distance map input from the distance information acquisition section 340. The distance information correction section 371 outputs the distance map thus corrected to the comparison section 373.

The depth-of-field acquisition section 372 is connected to the control section 302, and receives information about the zoom lens position from the control section 302. The zoom lens position is set using the zoom lever 501, and has the relationship described above with reference to FIG. 5 with the distance to the object at which the object is in focus, and the depth of field. The depth-of-field acquisition section 372 determines the in-focus range (i.e., the range of the distance to the object at which the object is in focus) using a look-up table or the like based on the information about the zoom lens position input from the control section 302, and outputs the in-focus range to the comparison section 373. The look-up table may be set in advance based on the characteristics of the objective lens system 203 and the objective lens system 204.

The comparison section 373 compares the distance map input from the distance information correction section 371 with the information about the in-focus range input from the depth-of-field acquisition section 372 on a pixel basis to determine whether or not the object is in focus on a pixel basis. The comparison section 373 outputs the in-focus determination results to the in-focus determination map output section 374.

The in-focus determination map output section 374 generates an in-focus determination map based on the in-focus determination results input from the comparison section, and outputs the in-focus determination map to the classification section 310. The in-focus determination map is a map in which "1" is assigned to a pixel for which it has been determined that the object is in focus, and "0" is assigned to a pixel for which it has been determined that the object is out of focus, for example. The in-focus determination map is data having the same size (i.e., the same number of pixels) as that of the image output from the image construction section 320.

The classification section 310 performs the classification process on each pixel (or each area) within the image based on the distance information and a classification reference. More specifically, the classification section 310 includes a surface shape calculation section 350 (three-dimensional shape calculation section) and a classification processing section 360. Note that the details of the classification process performed by the classification section 310 are described later. An outline of the classification process is described below.

The surface shape calculation section 350 calculates a normal vector to the surface of the object corresponding to each pixel of the distance map as surface shape information (three-dimensional shape information in a broad sense). The classification processing section 360 projects a reference pit pattern onto the surface of the object based on the normal vector. The classification processing section 360 adjusts the size of the reference pit pattern to the size within the image (i.e., an apparent size that decreases within the image as the distance increases) based on the distance at the corresponding pixel position. The classification processing section 360 performs the matching process on the corrected reference pit pattern and the image to detect an area that agrees with the reference pit pattern.

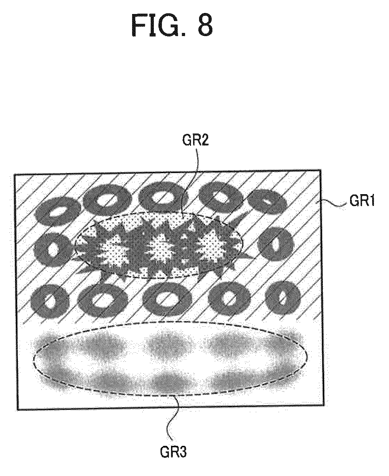

As illustrated in FIG. 8, the classification processing section 360 uses the shape of a normal pit pattern as the reference pit pattern, classifies an area GR1 that agrees with the reference pit pattern as "normal part", and classifies an area GR2 that does not agree with the reference pit pattern as "abnormal part (non-normal part or lesion)", for example. The classification processing section 360 corrects the classification results based on the results of the in-focus determination process. Specifically, the classification processing section 360 corrects the classification results for an area GR3 for which the in-focus determination section 370 has determined that the object is out of focus to "unknown". The classification processing section 360 may exclude a pixel for which it has been determined that the object is out of focus from the target of the matching process (i.e., classify the pixel as "unknown"), and performs the matching process on the remaining pixels to classify these pixels as "normal part" or "abnormal part". The classification processing section 360 outputs the classification results to the enhancement processing section 330.

Note that the classification "unknown" means that it is unknown whether to classify the structure of the object as "normal part" or "abnormal part" by the classification process that classifies the structure of the object corresponding to the type, the state (e.g., normal/abnormal), or the degree of abnormality of the structure. For example, when the structure of the object is classified as "normal part" or "abnormal part", the structure of the object that cannot be determined (that is not determined) to belong to "normal part" or "abnormal part" is classified as "unknown".

The enhancement processing section 330 performs the desired enhancement process on one image (e.g., the left image that is used as a reference when calculating the parallax) that forms the stereo image output from the image construction section 320 based on the classification results output from the classification section 310, and outputs the resulting image to the display section 400. Specifically, the enhancement processing section 330 does not output the stereo image, and the display section 400 displays a two-dimensional image. For example, the enhancement processing section 330 does not perform the enhancement process on the area GR1 that has been classified as "normal part", performs a luminance enhancement process on the area GR2 that has been classified as "abnormal part", and performs a process that replaces the pixel value with a specific color on the area GR3 that has been classified as "unknown". It is preferable that the specific color be a color that is not included in a normal object. When the user desires to observe the area that is displayed in the specific color, the user operates the zoom lever 501, or changes the relative distance between the imaging section 200 and the object so that the area is brought into focus. The user can thus obtain new classification results, and observe the area displayed in the specific color.

According to the first embodiment, the classification section 310 outputs a classification result (e.g., "unknown") that corresponds to an out-of-focus state (i.e., a state in which the object is out of focus) with respect to a pixel or an area for which it has been determined that the object is out of focus. Specifically, the classification section 310 corrects the result of the classification process to a classification that corresponds to the out-of-focus state with respect to the pixel or the area for which it has been determined that the object is out of focus.

According to this configuration, the classification results for an area of the image for which it has been determined that the object is out of focus are not output. Therefore, even when an unclear area of the image in which the object is out of focus has been erroneously classified as a classification that does not represent the actual state of the object, the unclear area is not enhanced (displayed in an enhanced state). This makes it possible to improve the reliability of enhancement display, and assist the user in diagnosis by presenting correct information to the user.

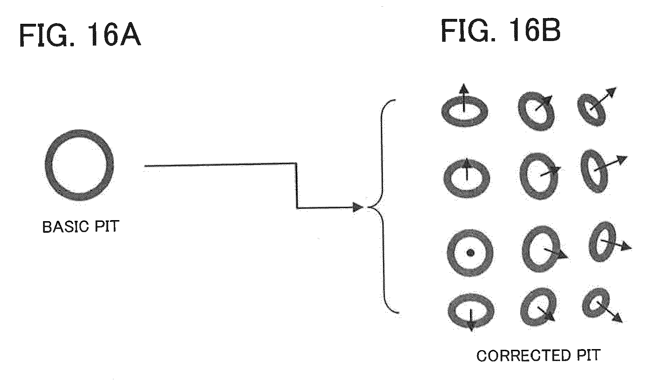

More specifically, the classification section 310 determines whether or not each pixel or each area agrees with the characteristics of a normal structure (e.g., the basic pit described later with reference to FIG. 16A) to classify each pixel or each area as a normal part or a non-normal part (abnormal part). The classification section 310 corrects the classification result that represents the normal part or the non-normal part to an unknown state with respect to the pixel or the area for which it has been determined that the object is out of focus, the unknown state representing that it is unknown whether the pixel or the area should be classified as the normal part or the non-normal part.

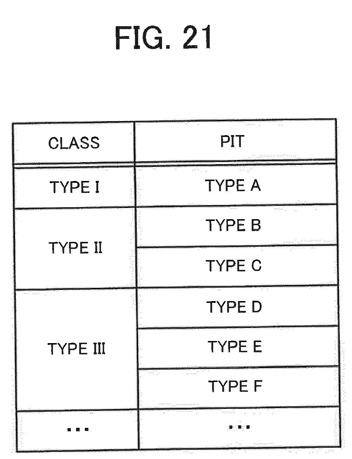

This makes it possible to classify the object as the normal part (e.g., a part in which a normal pit pattern is present) or the non-normal part other than the normal part, and suppress a situation in which an unclear area of the image in which the object is out of focus is erroneously classified as the non-normal part although a normal pit pattern is present. Note that the non-normal part may be subdivided (subclassified) as described later with reference to FIG. 21 and the like. In such a case, a situation may also occur in which the object is erroneously classified due to a motion blur. According to the first embodiment, however, it is possible to suppress such a situation.

The classification section 310 may exclude the pixel or the area for which it has been determined that the object is out of focus from the target of the classification process, and classify the pixel or the area as a classification that corresponds to the out-of-focus state.

In this case, since an area of the image in which the object is out of focus can be excluded from the target of the classification process, it is possible to suppress erroneous classification, and present correct information to the user. For example, it is possible to notify the user of an area that cannot be classified by setting the classification result for an area in which the object is out of focus to "unknown (unknown state)". Since the matching process is not performed on the pixel or the area for which it has been determined that the object is out of focus, the processing load can be reduced.

### 3. Second embodiment

### 3.1. Endoscope apparatus

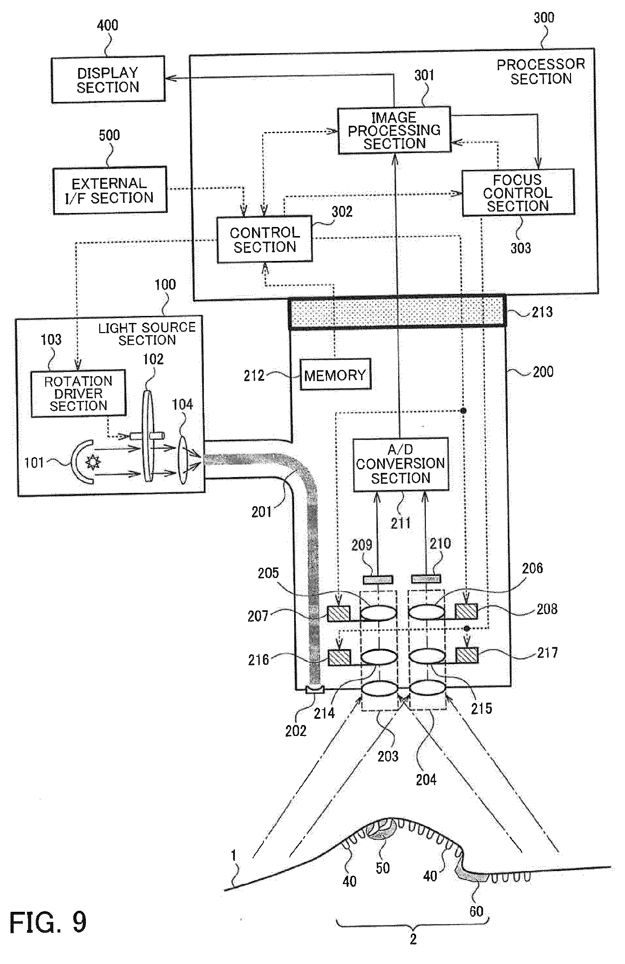

FIG. 9 illustrates a configuration example of an endoscope apparatus according to a second embodiment. The endoscope apparatus includes a light source section 100, an imaging section 200, a processor section 300, a display section 400, and an external I/F section 500. Note that the same elements as those described above in connection with the first embodiment are indicated by the same reference signs (symbols), and description thereof is appropriately omitted.

The endoscope apparatus according to the second embodiment differs from the endoscope apparatus according to the first embodiment as to the configuration of the objective lens system 203 and the objective lens system 204 included in the imaging section 200. Specifically, the objective lens system 203 further includes a focus lens 214, and the objective lens system 204 further includes a focus lens 215. The imaging section 200 further includes a focus lens driver section 216 that drives the focus lens 214, and a focus lens driver section 217 that drives the focus lens 215. The focus lens driver section 216 and the focus lens driver section 217 are implemented by a VCM, for example. The processor section 300 further includes a focus control section 303.

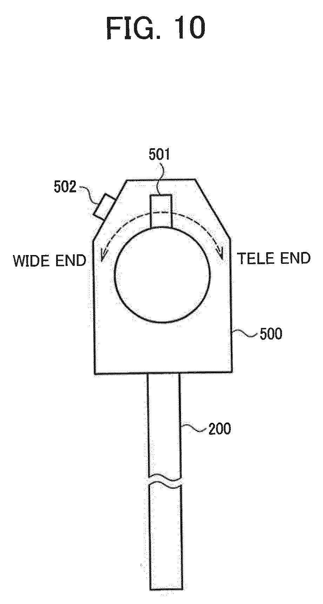

FIG. 10 illustrates a configuration example of the external I/F section 500 according to the second embodiment. The external I/F section 500 according to the second embodiment includes a zoom lever 501 and an AF button 502. The zoom lever 501 can be continuously operated within a given range. The user can continuously adjust the zoom lens position from the WIDE end to the TELE end by moving the zoom lever 501. The external I/F section 500 outputs position information about the zoom lever 501 to the control section 302. The external I/F section 500 outputs an AF start signal to the control section 302 when the AF button 502 has been pressed.

### 3.2. Focus control section

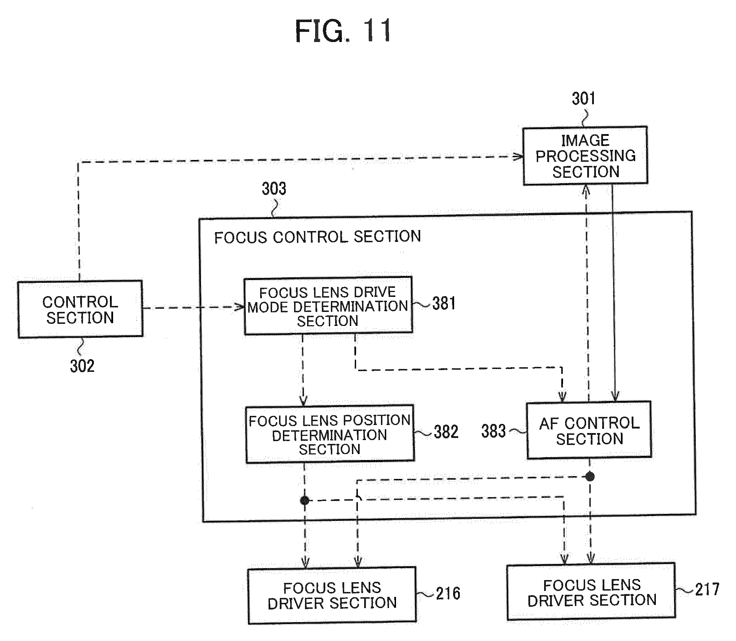

FIG. 11 illustrates a detailed configuration example of the focus control section 303. The focus control section 303 includes a focus lens drive mode determination section 381, a focus lens position determination section 382, and an AF (autofocus) control section 383.

The focus lens drive mode determination section 381 determines a focus lens drive mode based on information about the zoom lens position and AF start information input from the control section 302.

Specifically, the focus lens drive mode determination section 381 selects a fixed focus mode when the zoom lens is positioned on the WIDE side with respect to a given position, and outputs the information about the zoom lens position to the focus lens position determination section 382. The focus lens drive mode determination section 381 also selects the fixed focus mode when the zoom lens is positioned on the TELE side with respect to the given position, and the AF start signal is not input from the external I/F section 500, and outputs the information about the zoom lens position to the focus lens position determination section 382.

The focus lens position determination section 382 determines the focus lens position based on the information about the zoom lens position, and outputs information about the determined focus lens position to the focus lens driver section 216 and the focus lens driver section 217. Since the focus state changes when the zoom lens position has changed, a table in which the focus lens position that implements a fixed focus state is linked to each zoom lens position may be stored, and the focus lens position may be determined by referring to the table, for example. The focus lens driver section 216 and the focus lens driver section 217 respectively drive the focus lens 214 and the focus lens 215 based on the information about the focus lens position input from the focus lens position determination section 382.

The focus lens drive mode determination section 381 selects an AF mode when the zoom lens is positioned on the TELE side with respect to the given position, and the AF start signal has been input from the external I/F section 500, and outputs the AF start signal to the AF control section 383.

The AF control section 383 outputs an AF status signal that is set to a status "active" to the image processing section 301 when the AF start signal has been input from the focus lens drive mode determination section 381 to start AF operation. When the AF operation has started, the AF control section 383 calculates the contrast value from the image input from the image processing section 301, and drives the focus lens 214 and the focus lens 215 based on a known contrast AF method. In this case, the AF control section 383 outputs the information about the focus lens position to the image processing section 301 each time the AF control section 383 drives the focus lens 214 and the focus lens 215. The AF control section 383 determines whether or not an in-focus state has occurred from the calculated contrast value, and stops the AF operation when it has been determined that an in-focus state has occurred. The AF control section 383 then outputs the AF status signal that is set to a status "inactive" to the image processing section 301.

Note that the mode is switched between the fixed focus mode and the AF mode based on the zoom lens position since the depth of field differs depending on the zoom lens position (see FIG. 5). Specifically, when the zoom lens is positioned on the WIDE side, the AF control process is not required since the depth of field is sufficiently deep. On the other hand, when the zoom lens is positioned on the TELE side, the AF control process is required since the depth of field is shallow.

### 3.3. In-focus determination section

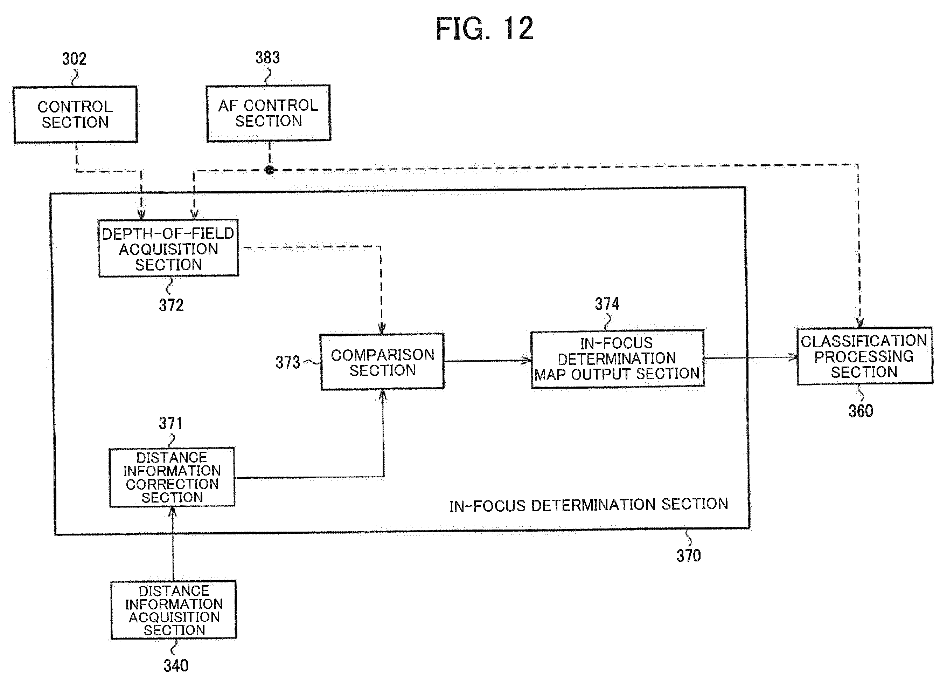

FIG. 12 illustrates a detailed configuration example of the in-focus determination section 370 according to the second embodiment. The in-focus determination section 370 includes a distance information correction section 371, a depth-of-field acquisition section 372, a comparison section 373, and an in-focus determination map output section 374.

The basic configuration of the in-focus determination section 370 is the same as described above in connection with the first embodiment. The in-focus determination section 370 according to the second embodiment differs from the in-focus determination section 370 according to the first embodiment in that the in-focus determination section 370 is connected to the control section 302 and the AF control section 383, and the depth-of-field acquisition section 372 operates in a way differing from that described above in connection with the first embodiment. Note that the depth-of-field acquisition section 372 operates in the same manner as described above in connection with the first embodiment when the AF status signal input from the AF control section 383 is set to "inactive" (i.e., fixed focus mode).

When the AF status signal is set to "active" (i.e., AF mode), the depth-of-field acquisition section 372 determines the in-focus range using a look-up table set in advance or the like based on the information about the zoom lens position input from the control section 302 and the information about the focus lens position input from the AF control section 383, and outputs the determined in-focus range to the comparison section 373.

### 3.4. Classification section

The classification processing section 360 according to the second embodiment is described below. The classification processing section 360 according to the second embodiment is connected to the AF control section 383. Note that the classification processing section 360 operates in the same manner as described above in connection with the first embodiment when the AF status signal input from the AF control section 383 is set to "inactive".

When the AF status signal is set to "active", the classification processing section 360 performs the matching process on the classification reference (that has been corrected based on the distance information) and the image to classify the object as "normal part" or "abnormal part", for example. The classification processing section 360 corrects classification based on the in-focus determination map input from the in-focus determination section 370. The classification processing section 360 stores a plurality of classification results and a plurality of in-focus determination maps during a period in which the AF status signal is set to "active". The classification processing section 360 determines one corrected classification based on the plurality of classification results and the plurality of in-focus determination maps. Specifically, the classification processing section 360 compares a plurality of in-focus maps, and uses the classification result when it has been determined that the object is in focus as the corrected classification with respect to a pixel for which it has been determined in the in-focus map that the object is in focus. The classification processing section 360 corrects the classification result to a classification "unknown" with respect to a pixel for which it has not been determined in each in-focus map that the object is in focus. The classification processing section 360 outputs the classification results in which each pixel is classified as "normal part", "abnormal part", or "unknown" to the enhancement processing section 330.

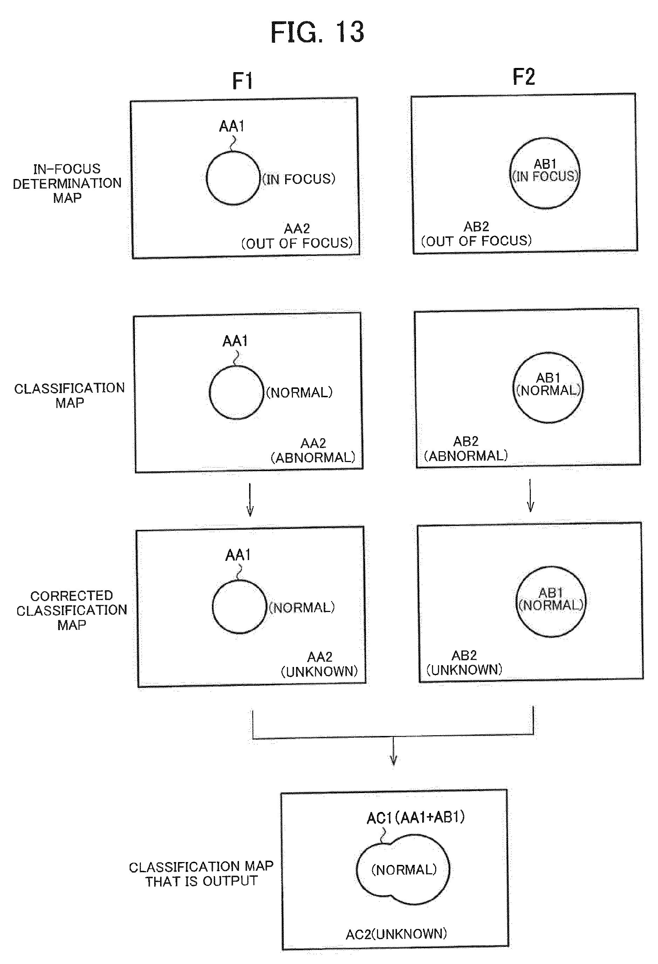

The operation of the classification processing section 360 is described below taking an example illustrated in FIG. 13 in which classification is corrected using the in-focus determination map that corresponds to a frame F1 and the in-focus determination map that corresponds to a frame F2. The frame F1 and the frame F2 are consecutive frames captured during the AF operation. Since the in-focus range changes due to the movement of the lens position during the AF operation, the in-focus determination map that corresponds to the frame F1 and the in-focus determination map that corresponds to the frame F2 differ in "in-focus" area.

In the in-focus determination map that corresponds to the frame F1, an area AA1 is determined to be an "in-focus" area, and an area AA2 other than the area AA1 is determined to be an "out-of-focus" area (see FIG. 13). In the classification map, the area AA1 is classified as "normal", and the area AA2 is classified as "abnormal" since the image is blurred. The classification map is corrected using the in-focus determination map so that the area AA2 is classified as "unknown". The classification map that corresponds to the frame F2 in which an area AB1 (i.e., "in-focus" area) is classified as "normal" is corrected so that an area AB2 (i.e., "out-of-focus" area) is classified as "unknown" instead of "abnormal". The classification processing section 360 compares the corrected classification map that corresponds to the frame F1 with the corrected classification map that corresponds to the frame F2. The classification processing section 360 classifies a pixel that is classified as "normal" in at least one of the corrected classification map that corresponds to the frame F1 and the corrected classification map that corresponds to the frame F2 as "normal", and classifies a pixel that is classified as "unknown" in both the corrected classification map that corresponds to the frame F1 and the corrected classification map that corresponds to the frame F2 as "unknown". An area AC1 obtained by combining the area AA1 in the frame F1 that is classified as "normal" and the area AB 1 in the frame F2 that is classified as "normal" is classified as "normal", and the final classification map is output.

According to the second embodiment, the AF control section 383 controls the autofocus operation of the imaging section 200. The in-focus determination section 370 determines whether or not the object is in focus in each of a plurality of frames (e.g., frame F1 and frame F2) in which the autofocus operation is performed. The classification section 310 outputs the result ("normal" (see the area AA1 and the area AB1 in FIG. 13)) of the classification process that corresponds to the frame in which it has been determined that the object is in focus as the final classification result ("normal" (see the area AC1)) with respect to a pixel or an area for which it has been determined that the object is in focus in the frame among the plurality of frames.

This makes it possible to output the final classification results using the information (in-focus determination map and classification map) acquired corresponding to a plurality of focus lens positions. Therefore, even when the depth of field is shallow (e.g., during zoom observation), the size of an area that is finally classified as "unknown" can be reduced by utilizing the fact that the in-focus range changes on a frame basis due to the AF operation. This makes it possible to display highly reliable classification results obtained within the in-focus area over a larger area.

### 4. First classification method

### 4.1. Classification section

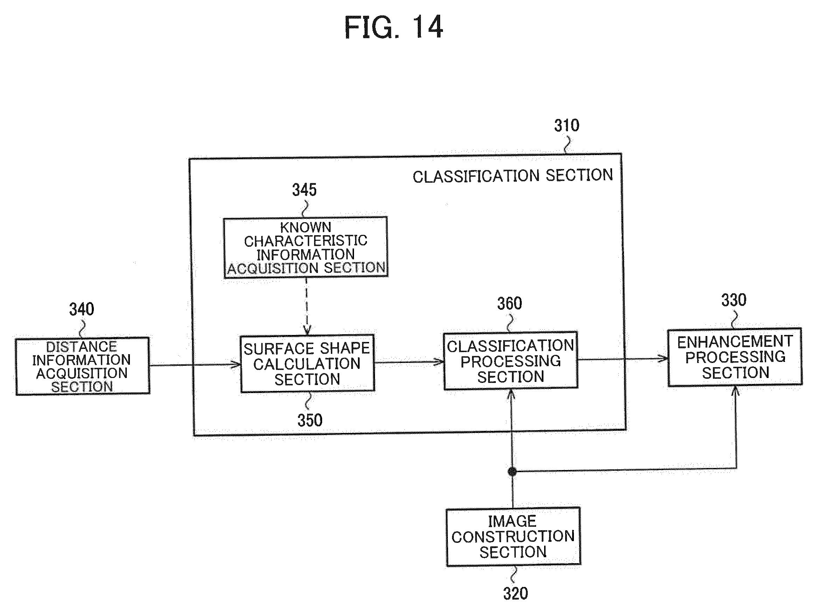

The classification process performed by the classification section 310 according to the first and second embodiments is described in detail below. FIG. 14 illustrates a detailed configuration example of the classification section 310. The classification section 310 includes a known characteristic information acquisition section 345, the surface shape calculation section 350, and the classification processing section 360.

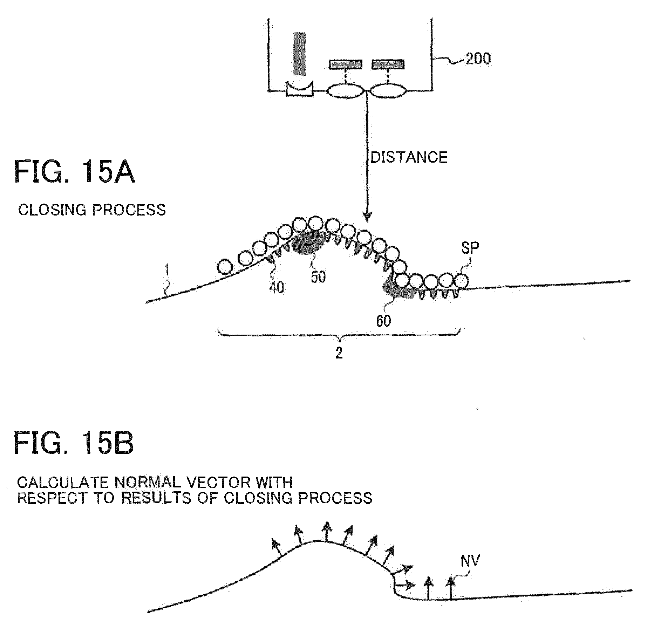

The operation of the classification section 310 is described below taking an example in which the observation target is the large intestine. As illustrated in FIG. 15A, a polyp 2 (i.e., elevated lesion) is present on the surface 1 of the large intestine (i.e., observation target), and a normal duct 40 and an abnormal duct 50 are present in the surface layer of the mucous membrane of the polyp 2. A recessed lesion 60 (in which the ductal structure has disappeared) is present at the base of the polyp 2. As illustrated in FIG. 1B, when the polyp 2 is viewed from above, the normal duct 40 has an approximately circular shape, and the abnormal duct 50 has a shape differing from that of the normal duct 40.

The surface shape calculation section 350 performs a closing process or an adaptive low-pass filtering process on the distance information (e.g., distance map) input from the distance information acquisition section 340 to extract a structure having a size equal to or larger than that of a given structural element. The given structural element is the classification target ductal structure (pit pattern) formed on the surface 1 of the observation target part.

Specifically, the known characteristic information acquisition section 345 acquires structural element information as the known characteristic information, and outputs the structural element information to the surface shape calculation section 350. The structural element information is size information that is determined by the optical magnification of the imaging section 200, and the size (width information) of the ductal structure to be classified from the surface structure of the surface 1. Specifically, the optical magnification is determined corresponding to the distance to the object, and the size of the ductal structure within the image captured at a specific distance to the object is acquired as the structural element information by performing a size adjustment process using the optical magnification.

For example, the control section 302 included in the processor section 300 stores a standard size of a ductal structure, and the known characteristic information acquisition section 345 acquires the standard size from the control section 302, and performs the size adjustment process using the optical magnification. Specifically, the control section 302 determines the observation target part based on the scope ID information input from the memory 212 included in the imaging section 200. For example, when the imaging section 200 is an upper gastrointestinal scope, the observation target part is determined to be the gullet, the stomach, or the duodenum. When the imaging section 200 is a lower gastrointestinal scope, the observation target part is determined to be the large intestine. A standard duct size corresponding to each observation target part is stored in the control section 302 in advance. When the external I/F section 500 includes a switch that can be operated by the user for selecting the observation target part, the user may select the observation target part by operating the switch, for example.

The surface shape calculation section 350 adaptively generates surface shape calculation information based on the input distance information, and calculates the surface shape information about the object using the surface shape calculation information. The surface shape information represents the normal vector NV illustrated in FIG. 15B, for example. The details of the surface shape calculation information are described later. For example, the surface shape calculation information may be the morphological kernel size (i.e., the size of the structural element) that is adapted to the distance information at the attention position on the distance map, or may be the low-pass characteristics of a filter that is adapted to the distance information. Specifically, the surface shape calculation information is information that adaptively changes the characteristics of a nonlinear or linear low-pass filter corresponding to the distance information.

The surface shape information thus generated is input to the classification processing section 360 together with the distance map. As illustrated in FIGS. 16A and 16B, the classification processing section 360 generates a corrected pit (classification reference) from a basic pit corresponding to the three-dimensional shape of the surface of tissue captured within the captured image. The basic pit is generated by modeling a normal ductal structure for classifying a ductal structure. The basic pit is a binary image, for example. The terms "basic pit" and "corrected pit" are used since the pit pattern is the classification target. Note that the terms "basic pit" and "corrected pit" can respectively be replaced by the terms "reference pattern" and "corrected pattern" having a broader meaning.

The classification processing section 360 performs the classification process using the generated classification reference (corrected pit). Specifically, the image output from the image construction section 320 is input to the classification processing section 360. The classification processing section 360 determines the presence or absence of the corrected pit within the captured image using a known pattern matching process, and outputs a classification map (in which the classification areas are grouped) to the enhancement processing section 330. The classification map is a map in which the captured image is classified into an area that includes the corrected pit and an area other than the area that includes the corrected pit. For example, the classification map is a binary image in which "1" is assigned to pixels included in an area that includes the corrected pit, and "0" is assigned to pixels included in an area other than the area that includes the corrected pit. When the object is classified as "unknown" corresponding to the in-focus determination results, "2" may be assigned to pixels included in an area that is classified as "unknown" (i.e., a ternary image may be used).

The image (having the same size as that of the classification image) output from the image construction section 320 is input to the enhancement processing section 330. The enhancement processing section 330 performs the enhancement process on the image output from the image construction section 320 using the information that represents the classification results.

### 4.2. Surface shape calculation section

The process performed by the surface shape calculation section 350 is described in detail below with reference to FIGS. 15A and 15B.

FIG. 15A is a cross-sectional view illustrating the surface 1 of the object and the imaging section 200 taken along the optical axis of the imaging section 200. FIG. 15A schematically illustrates a state in which the surface shape is calculated using the morphological process (closing process). The radius of a sphere SP (structural element) used for the closing process is set to be equal to or more than twice the size of the classification target ductal structure (surface shape calculation information), for example. The size of the ductal structure has been adjusted to the size within the image corresponding to the distance to the object corresponding to each pixel (see above).

It is possible to extract the three-dimensional surface shape of the smooth surface 1 without extracting the minute concavities and convexities of the normal duct 40, the abnormal duct 50, and the duct disappearance area 60 by utilizing the sphere SP having such a size. This makes it possible to reduce a correction error as compared with the case of correcting the basic pit using the surface shape in which the minute concavities and convexities remain.

FIG. 15B is a cross-sectional view illustrating the surface of tissue after the closing process has been performed. FIG. 15B illustrates the results of a normal vector (NV) calculation process performed on the surface of tissue. The normal vector NV is used as the surface shape information. Note that the surface shape information is not limited to the normal vector NV. The surface shape information may be the curved surface illustrated in FIG. 15B, or may be another piece of information that represents the surface shape.

The known characteristic information acquisition section 345 acquires the size (e.g., the width in the longitudinal direction) of the duct of tissue as the known characteristic information, and determines the radius (corresponding to the size of the duct within the image) of the sphere SP used for the closing process. In this case, the radius of the sphere SP is set to be larger than the size of the duct within the image. The surface shape calculation section 350 can extract the desired surface shape by performing the closing process using the sphere SP.

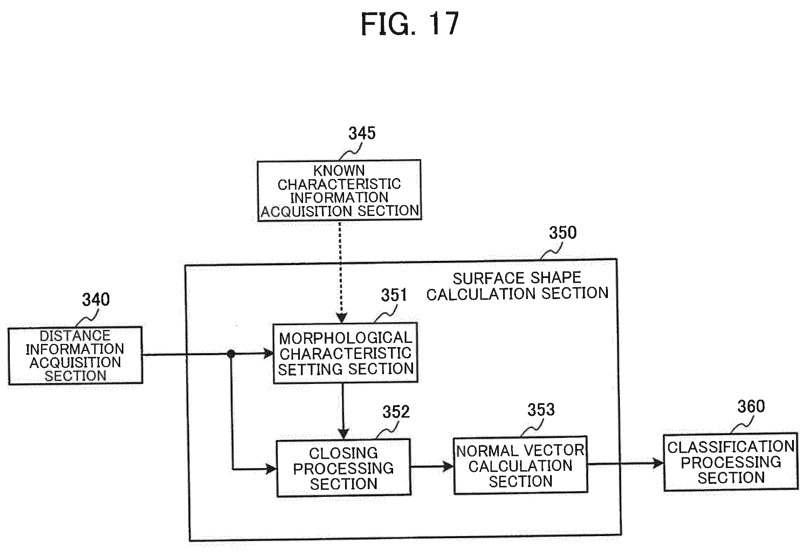

FIG. 17 illustrates a detailed configuration example of the surface shape calculation section 350. The surface shape calculation section 350 includes a morphological characteristic setting section 351, a closing processing section 352, and a normal vector calculation section 353.

The size (e.g., the width in the longitudinal direction) of the duct of tissue (i.e., known characteristic information) is input to the morphological characteristic setting section 351 from the known characteristic information acquisition section 345. The morphological characteristic setting section 351 determines the surface shape calculation information (e.g., the radius of the sphere SP used for the closing process) based on the size of the duct and the distance map.

The information about the radius of the sphere SP thus determined is input to the closing processing section 352 as a radius map having the same number of pixels as that of the distance map, for example. The radius map is a map in which the information about the radius of the sphere SP corresponding to each pixel is linked to each pixel. The closing processing section 352 performs the closing process while changing the radius of the sphere SP on a pixel basis using the radius map, and outputs the processing results to the normal vector calculation section 353.

The distance map obtained by the closing process is input to the normal vector calculation section 353. The normal vector calculation section 353 defines a plane using three-dimensional information (e.g., the coordinates of the pixel and the distance information at the corresponding coordinates) about the attention sampling position (sampling position in question) and two sampling positions adjacent thereto on the distance map, and calculates the normal vector to the defined plane. The normal vector calculation section 353 outputs the calculated normal vector to the classification processing section 360 as a normal vector map that is identical with the distance map as to the number of sampling points.

### 4.3. Classification processing section

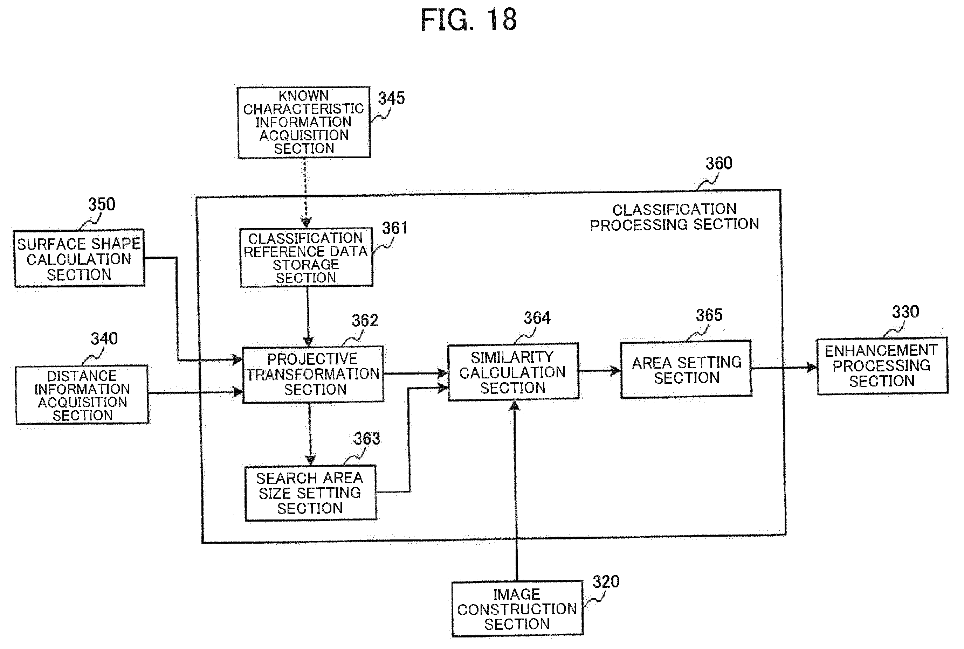

FIG. 18 illustrates a detailed configuration example of the classification processing section 360. The classification processing section 360 includes a classification reference data storage section 361, a projective transformation section 362, a search area size setting section 363, a similarity calculation section 364, and an area setting section 365.

The classification reference data storage section 361 stores the basic pit obtained by modeling the normal duct exposed on the surface of tissue (see FIG. 16A). The basic pit is a binary image having a size corresponding to the size of the normal duct captured at a given distance. The classification reference data storage section 361 outputs the basic pit to the projective transformation section 362.

The distance map output from the distance information acquisition section 340, the normal vector map output from the surface shape calculation section 350, and the optical magnification output from the control section 302 (not illustrated in FIG. 18) are input to the projective transformation section 362. The projective transformation section 362 extracts the distance information that corresponds to the attention sampling position from the distance map, and extracts the normal vector at the sampling position corresponding thereto from the normal vector map. The projective transformation section 362 subjects the basic pit to projective transformation using the normal vector, and performs a magnification correction process corresponding to the optical magnification to generate a corrected pit (see FIG. 16B). The projective transformation section 362 outputs the corrected pit to the similarity calculation section 36 as the classification reference, and outputs the size of the corrected pit to the search area size setting section 363.

The search area size setting section 363 sets an area having a size twice the size of the corrected pit to be a search area used for a similarity calculation process, and outputs the information about the search area to the similarity calculation section 364.

The similarity calculation section 364 receives the corrected pit at the attention sampling position from the projective transformation section 362, and receives the search area that corresponds to the corrected pit from the search area size setting section 363. The similarity calculation section 364 extracts the image of the search area from the image input from the image construction section 320.

The similarity calculation section 364 performs a high-pass filtering process or a band-pass filtering process on the extracted image of the search area to remove a low-frequency component, and performs a binarization process on the resulting image to generate a binary image of the search area. The similarity calculation section 364 performs a pattern matching process on the binary image of the search area using the corrected pit to calculate a correlation value, and outputs the peak position of the correlation value and a maximum correlation value map to the area setting section 365. The correlation value is the sum of absolute differences, and the maximum correlation value is the minimum value of the sum of absolute differences, for example.

Note that the correlation value may be calculated using a phase-only correlation (POC) method or the like. Since rotation and a change in magnification are invariable when using the POC method, it is possible to improve the correlation calculation accuracy.

The area setting section 365 calculates an area for which the sum of absolute differences is equal to or less than a given threshold value T based on the maximum correlation value map input from the similarity calculation section 364, and calculates the three-dimensional distance between the position within the calculated area that corresponds to the maximum correlation value and the position within the adjacent search range that corresponds to the maximum correlation value. When the calculated three-dimensional distance is included within a given error range, the area setting section 365 groups an area that includes the maximum correlation position as a normal part to generate a classification map. The area setting section 365 outputs the generated classification map to the enhancement processing section 330.

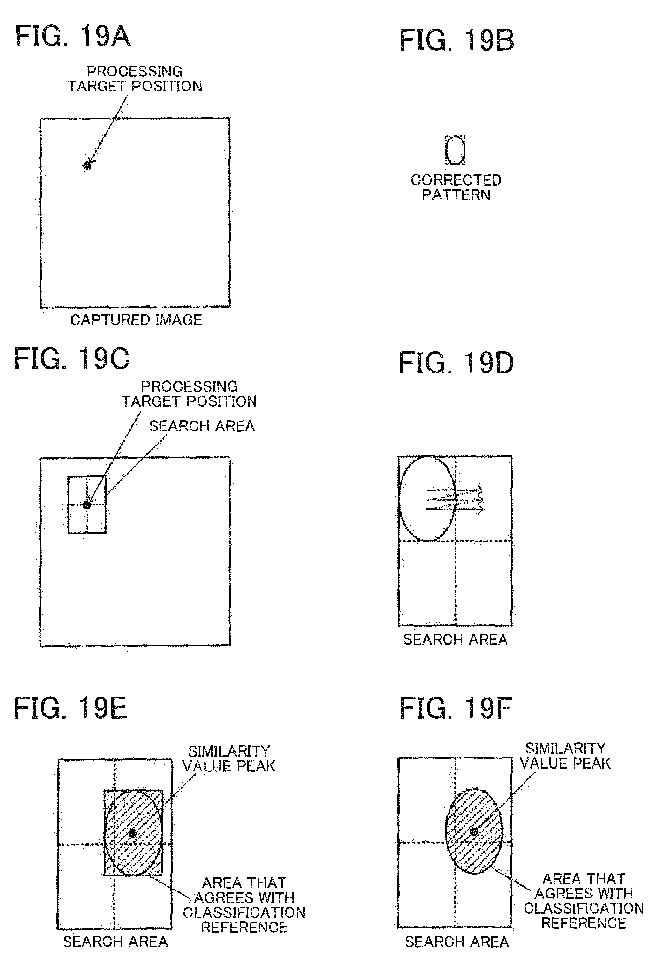

FIGS. 19A to 19F illustrate a specific example of the classification process. As illustrated in FIG. 19A, one position within the image is set to be the processing target position. The projective transformation section 362 acquires a corrected pattern at the processing target position by deforming the reference pattern based on the surface shape information that corresponds to the processing target position (see FIG. 19B). The search area size setting section 363 sets the search area (e.g., an area having a size twice the size of the corrected pit pattern) around the processing target position using the acquired corrected pattern (see FIG. 19C).

The similarity calculation section 364 performs the matching process on the captured structure and the corrected pattern within the search area (see FIG. 19D). When the matching process is performed on a pixel basis, the similarity is calculated on a pixel basis. The area setting section 365 determines a pixel that corresponds to the similarity peak within the search area (see FIG. 19E), and determines whether or not the similarity at the determined pixel is equal to or larger than a given threshold value. When the similarity at the determined pixel is equal to or larger than the threshold value (i.e., when the corrected pattern has been detected within the area having the size of the corrected pattern based on the peak position (the center of the corrected pattern is set to be the reference position in FIG. 19E)), it is determined that the area agrees with the reference pattern.

Note that the inside of the shape that represents the corrected pattern may be determined to be the area that agrees with the classification reference (see FIG. 19F). Various other modifications may also be made. When the similarity at the determined pixel is less than the threshold value, it is determined that a structure that agrees with the reference pattern is not present in the area around the processing target position. An area (0, 1, or a plurality of areas) that agrees with the reference pattern, and an area other than the area that agrees with the reference pattern are set within the captured image by performing the above process corresponding to each position within the image. When a plurality of areas agree with the reference pattern, overlapping areas and contiguous areas among the plurality of areas are integrated to obtain the final classification results. Note that the classification process based on the similarity described above is only an example. The classification process may be performed using another method. The similarity may be calculated using various known methods that calculate the similarity between images or the difference between images, and detailed description thereof is omitted.

According to the second embodiment, the classification section 310 includes the surface shape calculation section 350 that calculates the surface shape information about the object based on the distance information and the known characteristic information, and the classification processing section 360 that generates the classification reference based on the surface shape information, and performs the classification process that utilizes the generated classification reference.

This makes it possible to adaptively generate the classification reference based on the surface shape represented by surface shape information, and perform the classification process. A decrease in the accuracy of the classification process due to the surface shape may occur due to deformation of the structure within the captured image caused by the angle formed by the optical axis (optical axis direction) of the imaging section 200 and the surface of the object, for example. The method according to the second embodiment makes it possible to accurately perform the classification process even in such a situation.

The known characteristic information acquisition section 345 may acquire the reference pattern that corresponds to the structure of the object in a given state as the known characteristic information, and the classification processing section 360 may generate the corrected pattern as the classification reference, and perform the classification process using the generated classification reference, the corrected pattern being acquired by performing a deformation process based on the surface shape information on the reference pattern.

This makes it possible to accurately perform the classification process even when the structure of the object has been captured in a deformed state corresponding to the surface shape. Specifically, a circular ductal structure may be captured in a variously deformed state (see FIG. 1B, for example). It is possible to appropriately detect and classify the pit pattern even in a deformed area by generating an appropriate corrected pattern (corrected pit in FIG. 16B) from the reference pattern (basic pit in FIG. 16A) corresponding to the surface shape, and utilizing the generated corrected pattern as the classification reference.

The known characteristic information acquisition section 345 may acquire the reference pattern that corresponds to the structure of the object in a normal state as the known characteristic information.

This makes it possible to implement the classification process that classifies the captured image into a normal part and an abnormal part. The term "abnormal part" refers to an area that is suspected to be a lesion when using a medical endoscope, for example. Since it is considered that the user normally pays attention to such an area, a situation in which an area to which attention should be paid is missed can be suppressed by appropriately classifying the captured image, for example.

The object may include a global three-dimensional structure, and a local concave-convex structure that is more local than the global three-dimensional structure, and the surface shape calculation section 350 may calculate the surface shape information by extracting the global three-dimensional structure among the global three-dimensional structure and the local concave-convex structure included in the object from the distance information.

This makes it possible to calculate the surface shape information from the global structure when the structures of the object are classified into a global structure and a local structure. Deformation of the reference pattern within the captured image predominantly occurs due to a global structure that is larger than the reference pattern. Therefore, it is possible to accurately perform the classification process by calculating the surface shape information from the global three-dimensional structure.

### 5. Second classification method

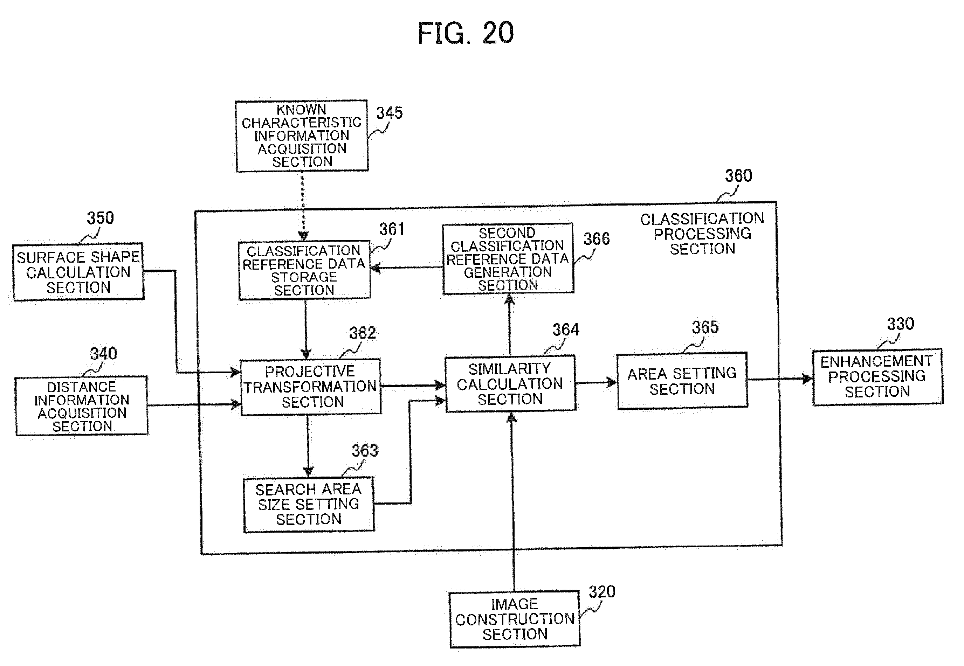

FIG. 20 illustrates a detailed configuration example of a classification processing section 360 that implements a second classification method. The classification processing section 360 includes a classification reference data storage section 361, a projective transformation section 362, a search area size setting section 363, a similarity calculation section 364, an area setting section 365, and a second classification reference data generation section 366. Note that the same elements as those described above in connection with the first classification method are indicated by the same reference signs (symbols), and description thereof is appropriately omitted.

The second classification method differs from the first classification method in that the basic pit (classification reference) is provided corresponding to the normal duct and the abnormal duct, a pit is extracted from the actual captured image, and used as second classification reference data (second reference pattern), and the similarity is calculated based on the second classification reference data.

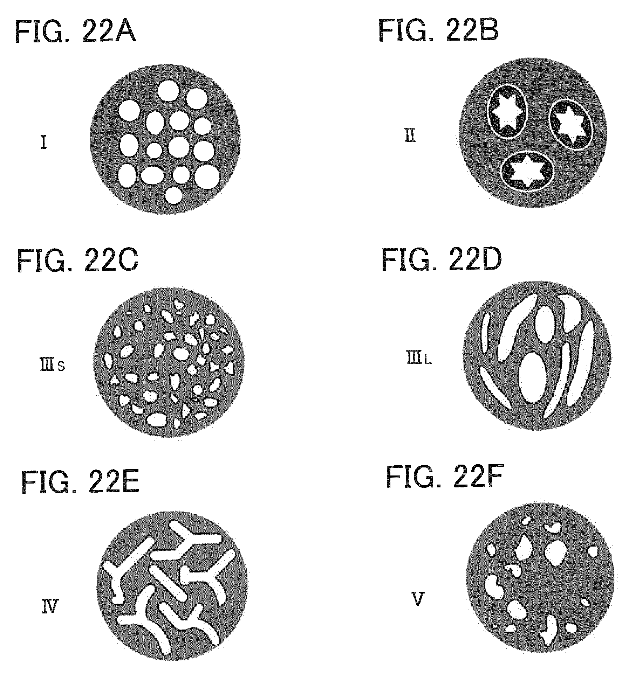

As illustrated in FIGS. 22A to 22F, the shape of a pit pattern on the surface of tissue changes corresponding to the state (normal state or abnormal state) of the pit pattern, the stage of lesion progression (when the state of the pit pattern is an abnormal state), and the like. For example, the pit pattern of a normal mucous membrane has an approximately circular shape (see FIG. 22A). The pit pattern has a complex shape (e.g., star-like shape (see FIG. 22B) or tubular shape (see FIGS. 22C and 22D)) when the lesion has advanced, and may disappear (see FIG. 22F) when the lesion has further advanced. Therefore, it is possible to determine the state of the object by storing these typical patterns as a reference pattern, and determining the similarity between the surface of the object captured within the captured image and the reference pattern, for example.

The differences from the first classification method are described in detail below. A plurality of pits including the basic pit corresponding to the normal duct (see FIG. 21) are stored in the classification reference data storage section 361, and output to the projective transformation section 362. The process performed by the projective transformation section 362 is the same as described above in connection with the first classification method. Specifically, the projective transformation section 362 performs the projective transformation process on each pit stored in the classification reference data storage section 361, and outputs the corrected pits corresponding to a plurality of classification types to the search area size setting section 363 and the similarity calculation section 364.

The similarity calculation section 364 generates the maximum correlation value map corresponding to each corrected pit. Note that the maximum correlation value map is not used to generate the classification map (i.e., the final output of the classification process), but is output to the second classification reference data generation section 366, and used to generate additional classification reference data.

The second classification reference data generation section 366 sets the pit image at a position within the image for which the similarity calculation section 364 has determined that the similarity is high (i.e., the absolute difference is equal to or smaller than a given threshold value) to be the classification reference. This makes it possible to implement a more optimum and accurate classification (determination) process since the pit extracted from the actual image is used as the classification reference instead of using a typical pit model provided in advance.

More specifically, the maximum correlation value map (corresponding to each type) output from the similarity calculation section 364, the image output from the image construction section 320, the distance map output from the distance information acquisition section 340, the optical magnification output from the control section 302, and the duct size (corresponding to each type) output from the known characteristic information acquisition section 345 are input to the second classification reference data generation section 366. The second classification reference data generation section 366 extracts the image data corresponding to the maximum correlation value sampling position (corresponding to each type) based on the distance information that corresponds to the maximum correlation value sampling position, the size of the duct, and the optical magnification.

The second classification reference data generation section 366 acquires a grayscale image (that cancels the difference in brightness) obtained by removing a low-frequency component from the extracted (actual) image, and outputs the grayscale image to the classification reference data storage section 361 as the second classification reference data together with the normal vector and the distance information. The classification reference data storage section 361 stores the second classification reference data and the relevant information. The second classification reference data having a high correlation with the object has thus been collected corresponding to each type.

Note that the second classification reference data includes the effects of the angle formed by the optical axis (optical axis direction) of the imaging section 200 and the surface of the object, and the effects of deformation (change in size) corresponding to the distance from the imaging section 200 to the surface of the object. The second classification reference data generation section 366 may generate the second classification reference data after performing a process that cancels these effects. Specifically, the results of the deformation process (projective transformation process and scaling process) performed on the grayscale image so as to achieve a state in which the image is captured at a given distance in a given reference direction may be used as the second classification reference data.

After the second classification reference data has been generated, the projective transformation section 362, the search area size setting section 363, and the similarity calculation section 364 perform the process on the second classification reference data. Specifically, the projective transformation process is performed on the second classification reference data to generate a second corrected pattern, and the process described above in connection with the first classification method is performed using the generated second corrected pattern as the classification reference.

Note that the basic pit corresponding to the abnormal duct used in connection with the second classification method is not normally point-symmetrical. Therefore, it is desirable that the similarity calculation section 364 calculate the similarity (when using the corrected pattern or the second corrected pattern) by performing a rotation-invariant phase-only correction (POC) process.

The area setting section 365 generates the classification map in which the pits are grouped on a class basis (type I, type II, ...) (see FIG. 21), or generates the classification map in which the pits are grouped on a type basis (type A, type B, ...) (see FIG. 21). Specifically, the area setting section 365 generates the classification map of an area in which a correlation is obtained by the corrected pit classified as the normal duct, and generates the classification map of an area in which a correlation is obtained by the corrected pit classified as the abnormal duct on a class basis or a type basis. The area setting section 365 synthesizes these classification maps to generate a synthesized classification map (multi-valued image). In this case, the overlapping area of the areas in which a correlation is obtained corresponding to each class may be set to be an unclassified area, or may be set to the type having a higher malignant level. The area setting section 365 outputs the synthesized classification map to the enhancement processing section 330.

The enhancement processing section 330 performs the luminance or color enhancement process based on the classification map (multi-valued image), for example.

According to the second embodiment, the known characteristic information acquisition section 345 acquires the reference pattern that corresponds to the structure of the object in an abnormal state as the known characteristic information.

This makes it possible to acquire a plurality of reference patterns (see FIG. 21), generate the classification reference using the plurality of reference patterns, and perform the classification process, for example. Specifically, the state of the object can be finely classified by performing the classification process using the typical patterns illustrated in FIGS. 22A to 22F as the reference pattern.

The known characteristic information acquisition section 345 may acquire the reference pattern that corresponds to the structure of the object in a given state as the known characteristic information, and the classification processing section 360 may perform the deformation process based on the surface shape information on the reference pattern to acquire the corrected pattern, calculate the similarity between the structure of the object captured within the captured image and the corrected pattern corresponding to each position within the captured image, and acquire a second reference pattern candidate based on the calculated similarity. The classification processing section 360 may generate the second reference pattern as a new reference pattern based on the acquired second reference pattern candidate and the surface shape information, perform the deformation process based on the surface shape information on the second reference pattern to generate the second corrected pattern as the classification reference, and perform the classification process using the generated classification reference.

This makes it possible to generate the second reference pattern based on the captured image, and perform the classification process using the second reference pattern. Since the classification reference can be generated from the object that is captured within the captured image, the classification reference sufficiently reflects the characteristics of the object (processing target), and it is possible to improve the accuracy of the classification process as compared with the case of directly using the reference pattern acquired as the known characteristic information.

### 6. Software

Although an example in which each section included in the image processing section 301 is implemented by hardware has been described above, the configuration is not limited thereto. For example, a CPU may perform the process of each section on an image acquired using an imaging device and the distance information. Specifically, the process of each section may be implemented by software by causing the CPU to execute a program. Alternatively, part of the process of each section may be implemented by software.

In this case, a program stored in an information storage medium is read, and executed by a processor (e.g., CPU). The information storage medium (computer-readable medium) stores a program, data, and the like. The information storage medium may be an arbitrary recording medium that records (stores) a program that can be read by a computer system, such as a portable physical medium (e.g., CD-ROM, USB memory, MO disk, DVD disk, flexible disk (FD), magnetooptical disk, or IC card), a stationary physical medium (e.g., HDD, RAM, or ROM) that is provided inside or outside a computer system, or a communication medium that temporarily stores a program during transmission (e.g., a public line connected through a modem, or a local area network or a wide area network to which another computer system or a server is connected).

Specifically, a program is recorded on the recording medium so that the program can be read by a computer. A computer system (i.e., a device that includes an operation section, a processing section, a storage section, and an output section) implements an image processing device by reading the program from the recording medium, and executing the program. Note that the program need not necessarily be executed by a computer system. The embodiments of the invention may similarly be applied to the case where another computer system or a server executes the program, or another computer system and a server execute the program in cooperation. Note that a method for operating or controlling an image processing device (image processing method) may be implemented by an image processing device (hardware), or may be implemented by causing a CPU to execute a program that describes the process of the method.

Although only some embodiments of the invention and the modifications thereof have been described in detail above, those skilled in the art will readily appreciate that many modifications are possible in the embodiments and the modifications thereof without materially departing from the novel teachings and advantages of the invention. A plurality of elements described in connection with the above embodiments and the modifications thereof may be appropriately combined to implement various configurations. For example, some elements may be omitted from the elements described in connection with the above embodiments and the modifications thereof. Some of the elements described above in connection with different embodiments or modifications thereof may be appropriately combined. Specifically, various modifications and applications are possible without materially departing from the novel teachings and advantages of the invention. Any term cited with a different term having a broader meaning or the same meaning at least once in the specification and the drawings can be replaced by the different term in any place in the specification and the drawings.

### REFERENCE SIGNS LIST

1: surface of tissue, 2: polyp, 40: normal duct, 50: abnormal duct, 60: duct disappearance area, 100: light source section, 101: white light source, 102: rotary color filter, 103: rotation driver section, 104: condenser lens, 200: imaging section, 201: light guide fiber, 202: illumination lens, 203, 204: objective lens system, 205, 206: zoom lens, 207, 208: zoom lens driver section, 209, 210: image sensor, 211: A/D conversion section, 212: memory, 213: connector, 214, 215: focus lens, 216, 217: focus lens driver section, 300: processor section, 301: image processing section, 302: control section, 303: focus control section, 305: image acquisition section, 310: classification section, 320: image construction section, 330: enhancement processing section, 340: distance information acquisition section, 345: known characteristic information acquisition section, 350: surface shape calculation section, 351: morphological characteristic setting section, 352: closing processing section, 353: normal vector calculation section, 360: classification processing section, 361: classification reference data storage section, 362: projective transformation section, 363: search area size setting section, 364: similarity calculation section, 365: area setting section, 366: classification reference data generation section, 370: in-focus determination section, 371: distance information correction section, 372: depth-of-field acquisition section, 373: comparison section, 374: in-focus determination map output section, 381: focus lens drive mode determination section, 382: focus lens position determination section, 383: control section, 400: display section, 500: external I/F section, 501: zoom lever, 502: AF button, DA, DF1 to DF4: depth of field, F1, F2: frame, GR1 to GR3: area, LP1 to LP4: zoom lens position, NV: normal vector, SP: sphere

## Claims

1. An image processing device comprising:
an image acquisition section that acquires a captured image that includes an image of an object;
a distance information acquisition section that acquires distance information based on a distance from an imaging section to the object when the imaging section captured the captured image;
an in-focus determination section that determines whether or not the object is in focus within a pixel or an area within the captured image based on the distance information;
a classification section that performs a classification process that classifies a structure of the object, and controls a target of the classification process corresponding to results of the determination as to whether or not the object is in focus within the pixel or the area; and
an enhancement processing section that performs an enhancement process on the captured image based on results of the classification process.

2. The image processing device as defined in claim 1,
the classification section outputting a classification result that corresponds to an out-of-focus state with respect to the pixel or the area for which it has been determined that the object is out of focus.

3. The image processing device as defined in claim 2,
the classification section correcting the result of the classification process to a classification that corresponds to the out-of-focus state with respect to the pixel or the area for which it has been determined that the object is out of focus.

4. The image processing device as defined in claim 3,
the classification section determining whether or not the pixel or the area agrees with characteristics of a normal structure to classify the pixel or the area as a normal part or a non-normal part, and correcting a classification result that represents the normal part or the non-normal part to an unknown state with respect to the pixel or the area for which it has been determined that the object is out of focus, the unknown state representing that it is unknown whether the pixel or the area should be classified as the normal part or the non-normal part.

5. The image processing device as defined in claim 2,
the classification section excluding the pixel or the area for which it has been determined that the object is out of focus from the target of the classification process, and classifying the pixel or the area as a classification that corresponds to the out-of-focus state.

6. The image processing device as defined in claim 5,
the classification section determining whether or not the pixel or the area agrees with characteristics of a normal structure to classify the pixel or the area as a normal part or a non-normal part, excluding the pixel or the area for which it has been determined that the object is out of focus from the target of the classification process that classifies the pixel or the area as the normal part or the non-normal part, and classifying the pixel or the area as an unknown state that represents that it is unknown whether the pixel or the area should be classified as the normal part or the non-normal part.

7. The image processing device as defined in claim 1, further comprising:
a depth-of-field acquisition section that acquires depth-of-field information about the imaging section; and
a comparison section that compares the distance information with the depth-of-field information,
the in-focus determination section determining whether or not the object is in focus within the pixel or the area based on a comparison result of the comparison section.

8. The image processing device as defined in claim 7,
the in-focus determination section determining that the object is in focus within the pixel or the area when the comparison result represents that the distance to the object within the pixel or the area that is represented by the distance information is within a depth of field that is represented by the depth-of-field information.

9. The image processing device as defined in claim 7, further comprising:
a focus control section that controls a position of a focus lens that is included in the imaging section,
the depth-of-field acquisition section acquiring the depth-of-field information that corresponds to the position of the focus lens.

10. The image processing device as defined in claim 9, further comprising:
a control section that controls a position of a zoom lens that is included in the imaging section,
the depth-of-field acquisition section acquiring the depth-of-field information that corresponds to a combination of the position of the zoom lens and the position of the focus lens.

11. The image processing device as defined in claim 7, further comprising:
a control section that controls a position of a zoom lens that is included in the imaging section,
the depth-of-field acquisition section acquiring the depth-of-field information that corresponds to the position of the zoom lens.

12. The image processing device as defined in claim 1, further comprising:
an AF control section that controls an autofocus operation that is performed by the imaging section,
the in-focus determination section determining whether or not the object is in focus in each of a plurality of frames in which the autofocus operation is performed, and
the classification section outputting the result of the classification process that corresponds to a frame among the plurality of frames in which it has been determined that the object is in focus as a final classification result with respect to the pixel or the area for which it has been determined that the object is in focus in the frame among the plurality of frames.

13. The image processing device as defined in claim 2,
the enhancement processing section enhancing the pixel or the area for which the classification section has output the classification result that corresponds to the out-of-focus state.

14. The image processing device as defined in claim 13,
the classification section determining whether or not the pixel or the area agrees with characteristics of a normal structure to classify the pixel or the area as a normal part or a non-normal part, and outputting an unknown state as the classification result that corresponds to the out-of-focus state, the unknown state representing that it is unknown whether the pixel or the area should be classified as the normal part or the non-normal part, and
the enhancement processing section enhancing the pixel or the area that has been classified as the unknown state by the classification section.

15. The image processing device as defined in claim 1,
the classification section determining whether or not the pixel or the area agrees with characteristics of a normal structure to classify the pixel or the area as a normal part or a non-normal part, and
the enhancement processing section enhancing the pixel or the area that has been classified as the non-normal part by the classification section.

16. The image processing device as defined in claim 1,
the classification section performing the classification process that classifies the structure of the object based on the distance information.

17. The image processing device as defined in claim 16, further comprising:
a known characteristic information acquisition section that acquires known characteristic information, the known characteristic information being information that represents known characteristics relating to a structure of the object,
the classification section including:
a surface shape calculation section that calculates surface shape information about the object based on the distance information and the known characteristic information; and
a classification processing section that generates a classification reference based on the surface shape information, and performs the classification process that utilizes the generated classification reference.

18. The image processing device as defined in claim 17,
the known characteristic information acquisition section acquiring a reference pattern that corresponds to the structure of the object in a given state as the known characteristic information, and
the classification processing section generating a corrected pattern as the classification reference, and performing the classification process using the generated classification reference, the corrected pattern being acquired by performing a deformation process based on the surface shape information on the reference pattern.

19. An endoscope apparatus comprising the image processing device as defined in any one of claims 1 to 18.

20. A program that causes a computer to perform steps of:
acquiring a captured image that includes an image of an object;
acquiring distance information based on a distance from an imaging section to the object when the imaging section captured the captured image;
determining whether or not the object is in focus within a pixel or an area within the captured image based on the distance information;
performing a classification process that classifies a structure of the object, and controlling a target of the classification process corresponding to results of the determination as to whether or not the object is in focus within the pixel or the area; and
performing an enhancement process on the captured image based on results of the classification process.

21. An image processing method comprising:
acquiring a captured image that includes an image of an object;
acquiring distance information based on a distance from an imaging section to the object when the imaging section captured the captured image;
determining whether or not the object is in focus within a pixel or an area within the captured image based on the distance information;
performing a classification process that classifies a structure of the object, and controlling a target of the classification process corresponding to results of the determination as to whether or not the object is in focus within the pixel or the area; and
performing an enhancement process on the captured image based on results of the classification process.
